# EUROPEAN PATENT SPECIFICATION

(11) **EP 1 594 834 B1**
(45) Date of publication and mention of the grant of the patent: **07.04.2010**
(21) Application number: 03815936.4
(22) Date of filing: 21.02.2003
(51) Int. Cl.: C07C 67/02, C11B 13/00, C07J 75/00

(54) **PROCESS FOR OBTAINING FATTY ACID ALKYL ESTERS, ROSIN ACIDS AND STEROLS FROM CRUDE TALL OIL**
VERFAHREN ZUR GEWINNUNG VON FETTSÄUREALKYLESTERN, HARZSÄUREN UND STERINEN AUS ROHTALLÖL
PROCEDE POUR OBTENIR DES ESTERS D'ALKYLE D'ACIDE GRAS, DES ACIDES RESINIQUES ET DES STEROLS A PARTIR DE TALL OIL BRUT

(43) Date of publication of application: 16.11.2005
(73) Proprietor: Cognis IP Management GmbH, 40589 Düsseldorf (DE)
(72) Inventor: SATO, Setsuo, Jardim Santa Maria, Jacarei, SP (BR); SOUSA SALES, Henrique Jorge, 12240-000 Sao Jose dos Campos, SP (BR); PELOGGIA, Hercules, 12320-520-Jacarei-SP (BR); KEMPERS, Peter, 41189 Mönchengladbach (DE); BOTH, Sabine, 41470 Neuss (DE); SCHORKEN, Ulrich, 40591-Düsseldorf (DE); WOLF, Thomas, 42781-Haan (DE)
(74) Representative: Reinhardt, Jürgen
(86) International application number: PCT/BR2003/000024
(87) International publication number: WO 2004/074233

(56) References cited:
- US-A- 3 804 819
- US-A- 6 107 456
- US-B1- 6 344 573
- US-B2- 6 414 111

## Description

### Brief description of the invention

The invention refers to a process for obtaining fatty acid alkyl esters, rosin acids and sterols from crude tall oil (CTO) which involves several esterification and distillation steps.

### State of the art

The use of sterols to take control of cholesterol level in human nutrition body industry might increase a lot the demand of non-GMO sterols, consequently a commercial separation process of sterols from Crude Tall Oil is highly interesting from the economical viewpoint, since this is one of the main source of sterols. Crude tall oil typically comes from the sulphate process employed in the manufacture of cellulose from wood. More particularly, the spent black liquor from the pulping process is concentrated until sodium salts (soaps) of various acids separate out and are skimmed off. The salts are acidified or decomposed with sulphuric acid so as to provide the crude tall oil.

Crude tall oil is refined mainly by vacuum distillation processes to separate the various compounds almost completely into rosin and fatty acid fractions. The current technology is based in distillation where the acids are fractionated in several columns. Using a first column to separate the more volatile fatty acids and rosin acids, from the less volatile materials, which include many of the unsaponifiable and neutral materials such as sterols and their esters. A second column is commonly designed to separate the more volatile fatty acids from the less volatile rosin acids. This process usually ends up with a bottom that is currently called, as "pitch" where sterols, heavy hydrocarbons, wax alcohols are the main substances. Commercially only fatty and rosin acids are produced. Pitch usually is used as a fuel. Due to the high distillation temperature there is significant sterols degradation. Also the most part of the free sterols are converted into esters. Tall oil pitch is a very viscous, dark product, which is rather difficult to handle. So far, there is no economic commercial process running to extract sterols from the pitch. From the state of the art a number of processes are known describing ways to extract sterols from CTO soaps using solvents and distillation processes prior to any acid splitting process, which theoretically could avoid sterols losses [US 6,107,456; US 6,414,111; US 6,344,573], however, these processes are characterised by a high technical afford and were not reduced into practice for economical reasons.

A method of separating sterols from crude tall oil, wherein the sterols are not destroyed in the process, would be a useful invention in the chemical preparation industry. Therefore, the objective of this invention is to find out an economic process to separate the three main crude tall oil (CTO) components, fatty acids or their esters, rosin acids, and sterols, to get commercial valuable to these products.

### Detailed description of the invention

What claimed is a process for obtaining fatty acid alkyl esters, rosin acids and sterols from crude tall oil (CTO), which is characterised by the following steps:
(a) reacting the free fatty acids present in the CTO with C₁-C₄ alcohols;
(b) separating the fatty acid lower C₁-C₄ alkyl esters thus obtained from the remaining CTO to produce a first stream of fatty acid esters;
(c) esterifying the sterols in the remaining CTO with boric acid;
(d) separating the remaining rosin acids from the sterol borates previously obtained to produce a second stream of rosin acids; and
(e) converting said sterol borates into the free sterols to produce a third stream of free sterols.

In more detail, in step (a) of the process the fatty acids are converted into their respective C₁-C₄ alkyl, preferably into their methyl esters. The major advantage of this step lies in the low boiling point of the esters thus obtained, what makes it easy to separate them from the other fractions. This is preferably done in a single esterification step due to the selective enzymatic or chemical reaction between fatty and rosin acids, with means that usually only the fatty acids are converted into their respective esters. The esterification can be conducted by means of acidic catalysts, like for example methane sulfonic acid at temperatures of 120 to 150 °C, or enzymes, like for example Novozym CaLB (Novo) at temperatures of 20 to 50 °C, depending on the activity optimum of the micro-organisms. Usually, the enzymatical reaction takes a significant longer time. The esterification can be carried out under pressure.

In step (b) the fatty acid esters thus obtained are separated from the remaining CTO by means of distillation, short path distillation or fractionation, employing milder conditions compared to the acid distillation with the advantage of getting fatty acid alkyl, preferably fatty acid methyl esters without rosin acids contamination and leaving less fatty acids in the bottom stream. The distillation is preferably carried out by means of a wiped film evaporator which is usually conducted at a reduced pressure of 1.33 - 1330 Pa (0.01 to 10 mmHg) and a temperature of 190 to 240 °C.

After the fatty acid methyl esters distillation boric acid (H₃BO₃ ) is added to the bottom stream which contains sterols and rosin acids to transform all the free sterols into sterols triesters (step c). Sterol esters are much more stable than free sterols itself which leads to less degradation products, specially the dehydration reaction. By this step it is possible to achieve a better separation between rosins and neutrals and to avoid the unwanted degradation of the sterols. Usually, the esterification is conducted a temperature of 200 to 230 °C.

Finally, according to steps (d) and (e) the rosin acids also are separated from the sterols borate esters and other high molecular weight hydrocarbons preferably by means of a short pass distillation, preferably by means of a wiped film evaporator, which is again operated preferably at a reduced pressure of from 1.33-1330 Pa (0.01 to 10 mmHg) and a temperature of 190 to 240 °C. After the rosin acids distillation the borate sterol esters are easily converted to the free sterols through hydrolysis or solvolysis. The preferred solvent, however, is water.

This process can also be applied to the tall oil pitch to enrich the sterols content. The borate esters step can be applied to separate tocopherols and sterols from the fatty acids portion in the soy bean vegetable oil distillate (VOD), and also to separate sterols and high molecular alcohols in the sugar cane waxes. The first stream, fatty acid methyl esters, is used to produce methyl dimerate, a raw material used to make polyamides as described in the US 6,281,373. The second stream, the rosin acids, is used to produce adhesives and other conventional products. The third stream, can be used as high sterols feed in the existent purification sterols processes. The viscosity of this stream can be decreased by adding soy bean oil during the last distillation or by reacting alcohols, C₁₂-C₁₈-saturated or unsaturated during the boric acid esterification step. The alcohols can be recovered after the hydrolysis step.

### Examples

### Comparative example C1

### Wiped film evaporator distillation of crude tall oil

This example illustrates for comparison purpose a wiped film evaporator (WFE) distillation of crude tall oil (CTO) in the same wiped film evaporator equipment used to develop the entire process, without selective enzymatic or chemical esterification of fatty acid and without transforms all free sterols into sterols borate triesters.

600,0 ml/h of CTO were passed through a WFE. The CTO contained 4,7 % b.w. sterol, of which only 9,0 % b.w. was already present as sterol esters. The WFE was operated at 1 mm Hg, with a initial residue temperature of 190 °C. The residue fraction ( residue 1 ) leaving the bottom of the WFE represented 64,0 % b.w. of the CTO feed. The residue 1 contained 38,0 % b.w. rosin acids, 40,0 b.w. % of TOFA. In the second distillation, the WFE was operated at 133 Pa (1 mm Hg), with a initial residue 1 temperature of 240 °C. The residue fraction ( residue 2 ) leaving the bottom of the WFE represented 15,0 % b.w. of the residue 1 feed. The residue 2 contained 40 % b.w. rosin acids, and 6 % b.w. total sterol. The sterol yield in this process was 25 % b.w., which means that 75 % b.w. of the sterols were degradated or distilled off together the rosins and fatty acids.

From this example one can see that is not possible to separate the fatty acids from the rosin acids using short path distillation and also the sterols recovery is too low.

### Inventive example 1

### Crude tall oil chemical esterification and distillation:

This inventive example describes the use of an selective chemical esterification of fatty acid from crude tall oil followed by the wiped film evaporator (WFE) to separate the fatty acids as methyl esters from the remaining heavy products, as used in accordance with the present invention, to avoid the sterols degradation in the residue fraction, and producing a high quality fatty acid ester (TOFA-Me) and rosin acids from crude tall oil.

### (a) Esterification step

1 kg of CTO obtained from RESITEC Indústrias Quimicas LTDA, were placed together with 750 g (23,43 moles) of methanol from Aldrich Chemical Co. and 12 g of methanesulfonic acid (0,12 moles) from Merck KGaA, into a 2-I-Büchi laboratory autoclave BEP 280 equipped with a thermometer, and mechanical agitator. Over a two-hour period, the temperature was maintained at 140°C, than the temperature was reduced from 140°C to 70°C and the unreacted methanol distilled off. The maximum reaction pressure in the reaction was 7 bar. The acid value of the CTO was reduced from initially 154 mgKOH/g to 65 mgKOH/g.

### (b) Distillation step

1,0 kg/h of CTO were passed through a WFE. The initial CTO contained 4,7 % b.w. sterol, of which only 9,0 % b.w. were already present as sterol esters, 40.2 % b.w. of rosin acids and 45 % b.w. of fatty acids. The WFE were operated at 133 Pa (1 mm Hg), with a initial residue temperature of 190 °C. The residue fraction ( residue 1 ) leaving the bottom of the WFE represented 55 % b.w. of the CTO feed having 8.6 % b.w. total sterols. The sterol yield in this first distillation has been 99.4 % b.w.. The remaining 0.6 % b.w. of the sterols was evaporated along the distillate 1 which had 93 % b.w. of TOFA-Me and 7 % b.w. of light boilers. Thermal degradation reactions are minimal. Subsequently, to 1 kg of residue 1 5,0 g of boric acid ( 0,08 moles ) obtained from Aldrich Chemical Co., were added. The sample was transferred into a 2-liter, 3-nechked round bottom flask for 4 h at 220°C to bond all the free sterols into esters. After this step the product was distilled through the WFE which was operated at 133 Pa (1 mm Hg), with a residue temperature of 240 °C. The residue 2 fraction leaving the bottom of the WFE represented 35% of the CTO feed. The residue 2 had 21 % b.w. total sterols. The total sterols yield in the entire process ( distillation 1 and 2) was 88 % of the theory.

### Inventive example 2

### Crude Tall Oil enzymatic esterification and distillation:

This example describes the use of an selective enzymatic esterification of fatty acid from crude tall oil and a wiped film evaporator (WFE), as used in accordance with the present invention, to increase the yield of sterols in the residue fraction, and to produce a high quality fatty acid ester (TOFA-Me = tall oil fatty acid methyl esters) and rosinic acid from crude tall oil.

### (a) Esterification step

3 kg of CTO obtained from RESITEC Indústrias Químicas LTDA were placed together with 750 g ( 23,43 moles) of methanol from Aldrich Chemical Co., 120 g of water, and 1,5 g of Novozym CaLB L from NOVO-ZYMES Latin America Ltda, in a 4-I-3-necked round bottom flask equipped with a thermometer, a mechanical agitator, and a condenser. The samples were shaken for 180 hrs at 30°C. The initial acid value of 154,0 mgKOH/g was reduced to 64,0 mgKOH/g.

### (b) Distillation step

After enzymatic esterification the product was distilled in the WFE which was operated at 133 Pa (1 mm Hg), with a initial residue temperature of 190°C. The residue 1 leaving the bottom of the WFE represented 54,0 % b.w. of the CTO feed. The residue 1 contained 8,8 % b.w. total sterols. The sterols yield in this first fractionation represented 99,4 % b.w. The remaining 0,6 % b.w. of the sterol were evaporated along with the volatile portion of the CTO. Thermal degradation reactions were minimal. Subsequently, to 1 kg of residue 1 5,0 g of boric acid ( 0,08 moles ) obtained from Aldrich Chemical Co., the sample were added. The sample was transferred into a 2-liter, 3-nechked round bottom flask for 4 h at 220 °C to bond all the free sterols into esters. After the reaction the product was distilled through a WFE operating at 133 Pa (1 mm Hg) with a residue temperature of 240 °C. The residue 2 fraction leaving the bottom of the WFE represented 35,0 % b.w. of the residue 1 feed having 21 % b.w. of sterols. Thermal degradation products were 6,0 % b.w. in the total.

## Claims

1. A process for obtaining fatty acid alkyl esters, rosin acids and sterols from crude tall oil (CTO), **characterised in that** said CTO is subjected to the following steps:
(a) reacting the free fatty acids present in the CTO with C₁-C₄ alcohols;
(b) separating the fatty acid C₁-C₄ alkyl esters thus obtained from the remaining CTO to produce a first stream of fatty acid esters;
(c) esterifying the sterols in the remaining CTO with boric acid;
(d) separating the remaining rosin acids from the sterol borates previously obtained to produce a second stream of rosin acids; and
(e) converting said sterol borates into the free sterols to produce a third stream of free sterols.

2. Process according to claim 1, **characterised in that** with respect to step (a) the esterification is carried out in the presence of acidic catalysts.

3. Process according to claim 2, **characterised in that** methane sulfonic acid is used as the catalyst.

4. Process according to claim 2 and/or 3, **characterised in that** the esterification is conducted at a temperature of 120 to 150 °C.

5. Process according to claim 1, **characterised in that** with respect to step (a) the esterification is carried out in the presence of enzymes.

6. Process according to claim 5, **characterised in that** the esterification is conducted at a temperature of 20 to 50 °C.

7. Process according to any of claims 1 to 6, **characterised in that** with respect to steps (b) and (d) the distillation is carried out by means of a wiped film evaporator.

8. Process according to claim 7, **characterised in that** said wiped film evaporator is operated at a reduced pressure of 1.33-1330 Pa (0.01 to 10 mm/Hg) and a temperature of 190 to 240 °C.

9. Process according to any of claims 1 to 8, **characterised in that** with respect to step (c) the esterification is conducted at a temperature of 200 to 230 °C.

10. Process according to any of claims 1 to 9, **characterised in that** with respect to step (e) the solvolysis of the sterol borates is affected by means of water.

## Patentansprüche

1. Verfahren zur Gewinnung von Fettsäurealkylestern, Harzsäure und Sterolen aus Rohtallöl, **dadurch gekennzeichnet, dass** man das Rohtallöl folgenden Schritten unterwirft:
(a) Umsetzung der im Rohtallöl vorliegenden freien Fettsäuren mit C₁-C₄ niederen Alkoholen;
(b) Abtrennung der so erhaltenen Fettsäurealkylester vom verbleibenden Rohtallöl, zur Herstellung eines Stoffstroms von Fettsäureestern;
(c) Veresterung der im Rohtallöl verbliebenen Sterole mit Borsäure;
(d) Trennung zurückgebliebenen Harzsäuren von den so erhaltenen Sterolboraten, zur Herstellung eines zweiten Stoffstroms von Harzsäuren, und
(e) Umwandlung der Sterolborate in freie Sterole, zur Herstellung eines dritten Stoffstroms von freien Sterolen.

2. Verfahren nach Anspruch 1, **dadurch gekennzeichnet, dass** man die Veresterung im Schritt (a) in Gegenwart von sauren Katalysatoren durchführt.

3. Verfahren nach Anspruch 2, **dadurch gekennzeichnet, dass** man als Katalysator Methansulfonsäure verwendet.

4. Verfahren nach Anspruch 2 und/oder 3, **dadurch gekennzeichnet, dass** man die Veresterung bei einer Temperatur von 120 bis 150°C durchführt.

5. Verfahren nach Anspruch 1, **dadurch gekennzeichnet, dass** man die Veresterung im Schritt (a) in Gegenwart von Enzymen durchführt.

6. Verfahren nach Anspruch 5, **dadurch gekennzeichnet, dass** man die Veresterung bei einer Temperatur von 20 bis 50°C durchführt.

7. Verfahren nach einem der Ansprüche 1 bis 6, **dadurch gekennzeichnet, dass** man die Destillation der Schritte (b) und (d) mit Hilfe eines Dünnfilmverdampfers durchführt.

8. Verfahren nach Anspruch 7, **dadurch gekennzeichnet, dass** man den Dünnfilmverdampfer bei einem verminderten Druck von 0,01 bis 10 mmHg und einer Temperatur von 190 bis 240°C betreibt.

9. Verfahren nach einem der Ansprüche 1 bis 8, **dadurch gekennzeichnet, dass** die Veresterung im Schritt (c) bei einer Temperatur von 200-230°C durchgeführt wird.

10. Verfahren nach einem der Ansprüche 1 bis 9, **dadurch gekennzeichnet, daß** man die Hydrolyse der Sterolborate im Schritt (e) mit Hilfe von Wasser durchführt.

## Revendications

1. Procédé d'obtention d'esters alkyliques d'acides gras, d'acides de colophane et de stérols à partir de tallol brut (CTO, *crude tall oil*), **caractérisé en ce que** ledit CTO est soumis aux étapes suivantes :
(a) réaction des acides gras libres présents dans le CTO avec des alcools courts en C1-C4 ;
(b) séparation des esters alkyliques courts d'acides gras ainsi obtenus à partir du CTO restant pour obtenir un premier courant d'esters d'acides gras ;
(c) estérification des stérols dans le CTO restant à l'acide borique ;
(d) séparation des acides de colophane restants des borates de stérol précédemment obtenus pour obtenir un deuxième courant d'acides de colophane ; et
(e) conversion desdits borates de stérol en les stérols libres pour obtenir un troisième courant de stérols libres.

2. Procédé conforme à la revendication 1, **caractérisé en ce que**, dans l'étape (a), l'estérification est mise en oeuvre en présence de catalyseurs acides.

3. Procédé conforme à la revendication 2, **caractérisé en ce que** le catalyseur employé est l'acide méthanesulfonique.

4. Procédé conforme à la revendication 2 et/ou à la revendication 3, **caractérisé en ce que** l'estérification est mise en oeuvre à une température comprise entre 120 et 150 °C.

5. Procédé conforme à la revendication 1, **caractérisé en ce que**, dans l'étape (a), l'estérification est mise en oeuvre en présence d'enzymes.

6. Procédé conforme à la revendication 5, **caractérisé en ce que** l'estérification est mise en oeuvre à une température comprise entre 20 et 50 °C.

7. Procédé conforme à l'une quelconque des revendications 1 à 6, **caractérisé en ce que**, dans les étapes (b) et (d), la distillation est mise en oeuvre au moyen d'un évaporateur à film raclé.

8. Procédé conforme à la revendication 7, **caractérisé en ce que** ledit évaporateur à film raclé est utilisé à une pression réduite comprise entre 0,01 et 10 mm/Hg et à une température comprise entre 190 et 240 °C.

9. Procédé conforme à l'une quelconque des revendications 1 à 8, **caractérisé en ce que**, dans l'étape (c), l'estérification est mise en oeuvre à une température comprise entre 200 et 230 °C.

10. Procédé conforme à l'une quelconque des revendications 1 à 9, **caractérisé en ce que**, dans l'étape (e), la solvolyse des borates de stérol est effectuée avec de l'eau.
